# EUROPEAN PATENT APPLICATION

(11) **EP 2 198 879 A1**
(43) Date of publication of application: **23.06.2010**
(21) Application number: 08171411.5
(22) Date of filing: 11.12.2008
(51) Int. Cl.: A61K 38/17, A61K 38/19, A61K 31/00, C07K 16/28, C12N 15/11, A61P 35/00, A61P 37/00, A61P 31/12, B01L 3/00

(54) **CD74 modulator agent for regulating dendritic cell migration and device for studying the motility capacity of a cell**

(71) Applicant: Institut Curie, 75005 Paris (FR); Centre National de la Recherche Scientifique- CNRS, 75016 Paris (FR); INSTITUT NATIONAL DE LA SANTE ET DE LA RECHERCHE MEDICALE (INSERM), 75013 Paris (FR)
(72) Inventor: Lennon-Dumenil, Ana-Maria, 75013 Paris (FR); Piel, Matthieu, 75012 Paris (FR)
(74) Representative: Gallois, Valérie

(57) **Abstract**

The present invention relates to a method for regulating the dendritic cell motility and/or migration *in vivo* using a CD74 modulator agent. It also concerns a device for determining or studying the motility capacity of a cell and the use thereof.
The CD74 modulator agent is of use in the treatment of auto-immune diseases, cancers, inflammatory diseases or viral infections.

## Description

### FIELD OF THE INVENTION

The present invention relates to the medicine field, in particular the immuno-regulation system. In particular, the present invention relates to a method for regulating the dendritic cell motility and/or velocity. The present invention also relates to methods and means to assay the motility and/or velocity of eukaryotic cells.

### BACKGROUND OF THE INVENTION

Dendritic Cells (DCs) patrol the body periphery by coupling efficient movement across tissues to high antigen (Ag) internalization capacity. Ag are internalized into endosomes where they are degraded into peptides that are loaded onto MHC molecules, resulting in the formation of MHC-peptide complexes that are subsequently exposed at the cell surface of DCs (1). If DCs detect an Ag-associated activating signal, they enter into a maturation program that comprises multiple changes among which: (1) an increase followed by a complete arrest in macropinocytosis and phagocytosis, maximizing the uptake and processing of the activating Ag (2-4), and (2) the modification of their migratory properties, enabling them to home to secondary lymphoid organs and present the resulting peptide-MHC complexes to T cells (5). Ag uptake/processing and cell migration must therefore be coordinated for DCs to efficiently activate T lymphocytes, suggesting that common regulatory mechanisms are involved. Although little is known on regulation of DC locomotion, it was recently shown that DCs use amoeboid-like migration to move in the interstitial spaces of tissues, a locomotion mode that relies on actomyosin contractility and occurs independently of Integrin-mediated adhesion (6).

The success of the immune response thus relies on the ability of immune cells to adapt their migratory capacity to external signals in order to be able to efficiently perform their effector function in response to changes in their microenvironment. How the specific function of immune cells is coordinated with cell migration is an essential question to be addressed. Accordingly, it could be useful to provide means for regulating DCs motility and migration.

One of the important cell behaviors for which the gap between in vivo and in vitro micro-environment differs the most obviously is cell migration. Cell migration has been studied for years and with a great success, on flat glass substrates. This simple set up well suited for microscopy has enabled the description of many important molecular mechanisms. But recent studies showed that it is certainly not the complete picture. Indeed, the cell environment in a living tissue is highly complex, involving cell/cell interactions as well as cell/matrix interactions. It has been shown recently using dendritic cells (DCs) from mutant mice that motility mechanisms can depend specifically on the geometry and physical properties of the cellular microenvironment. It was observed that integrin-deficient DCs move normally in tissues but are unable to produce motion on planar substrates. This example reveals the limitations of studying cell motility only on flat 2-D substrates and stresses the need for further strategies to understand the mechanisms of cell migration in tissue. Systems such as collagen gels are commonly used as simplified models to study cell migration in 3-D environments. However, even this very simplified system remains highly complex and results are thus difficult to interpret: many physical parameters act simultaneously and affect the motility of cells embedded in such gels. These parameters include not only geometrical confinement, which will depend on gel density, but also local constrictions, which will force cells to temporally squeeze their nucleus to pass through narrow spaces, as well as gel unevenness (rugosity) or elasticity (ability to deform); in collagen gels these parameters are all interwoven and their specific role on motility cannot be extracted. In this context, physical models are powerful tools since they enable to identify the minimal ingredients necessary for cell migration. From a physical point of view the cell is a striking example of "soft" system of micron size that can move autonomously. Identifying the basic mechanisms of self-motion of such systems is a major challenge that has raised a growing interest in recent years in the physics community.

### SUMMARY OF THE INVENTION

In a first aspect of the present invention, the inventors identified the MHCII-associated Invariant Chan (also called CD74 or Li) as a key factor for regulating the motility and the migration of dendritic cells (DCs).

Accordingly, the present invention relates to methods and means for modulating the capacity of DCs to migrate. Therefore, the present invention concerns a CD74 modulator agent for regulating the dendritic cell migration. In particular, the CD74 modulator agent is a CD74 inhibitor for increasing the dendritic cell migration. The CD74 inhibitor is preferably selected from the group consisting of a MIF inhibitor, an antibody against CD74, a CD74 antagonist, a soluble CD74, and an antisense, siRNA, shRNA and the like decreasing the expression of CD74. Alternatively, the CD74 modulator agent is a CD74 activator for decreasing the dendritic cell migration. The CD74 activator is selected from the group consisting of a CatS inhibitor, a CD74 agonist, MIF and a MIF agonist. In a particular embodiment, the CD74 modulator agent according to the present invention is useful for treating or preventing a tumor or cancer, an auto-immune disease, or atherosclerosis.

The present invention also concerns a device for determining or studying the motility capacity of a cell, wherein the device comprises at least one embedded micro-channel with holes for the inlet and outlet ports, said micro-channel having diameter smaller than the diameter of the studied cell. The present invention also concerns the use of the device according to the present invention for determining or studying the motility capacity of a cell. In particular, the present invention concerns a method for determining or studying the motility capacity of a cell, comprising introducing the cell into the inlet port of the device of the present invention and measuring the move of the cell into the micro-channel.

### BRIEF DESCRIPTION OF THE DRAWINGS

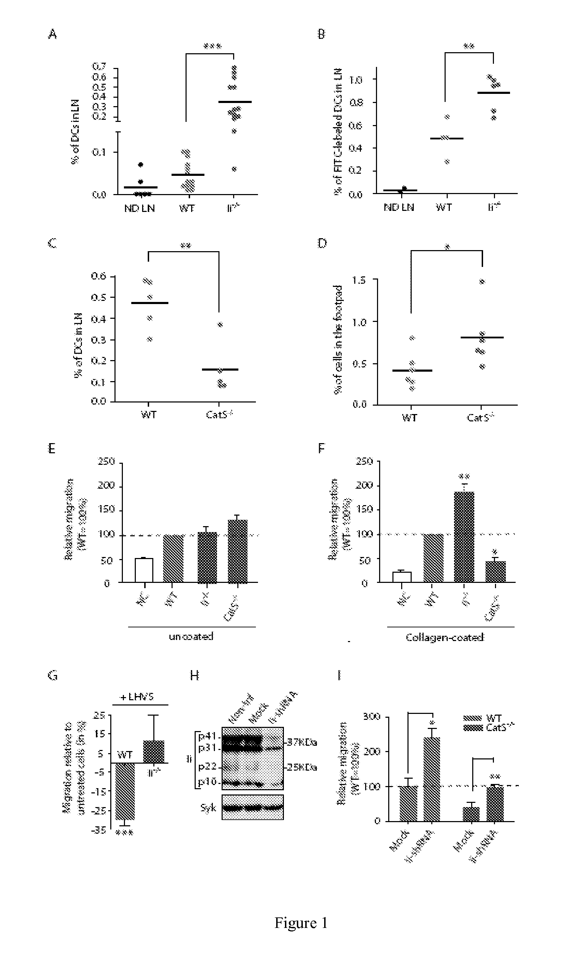

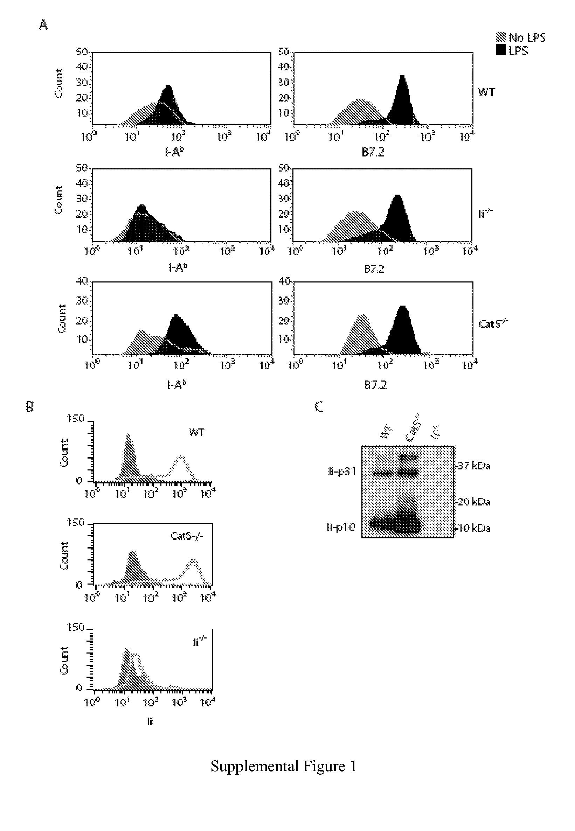

### Figure 1. Ii negatively regulates DC migration in vivo and in collagen-coated transwells.

**A:** WT and Ii^{-/-} DCs (3.10⁶) were labelled with CMTMR- and CFSE respectively, pre-treated with LPS (10µg/ml, 10min) and co-injected into the footpad of C57BL/6 recipient mice. 48h later, the draining and non-draining (ND) popliteal LN were collected, digested with collagenase D and their content analyzed by flow cytometry for the presence of fluorescent migrating DCs (in percentage of total LN cells, each dot represents one mouse, four independent experiments, ***, p<0,0001).
**B:** Ears from WT or Ii^{-/-} were painted with an irritating solution (dibutylphtalate plus acetone 50:50) containing 1% FITC. 24h later, draining LNs were collected, digested with collagenase D and analyzed for the presence of FITC+/CD11c+ cells by flow cytometry (each dot represent one mouse, three independent experiments **, p=0,004).
**C:** Migration of transferred WT and CatS^{-/-} BM-DCs in the footpad of C57BL/6 recipient mice. The experiment was performed as described in A.
**D:** the footpad of recipient mice where DCs were injected was dissected when collecting LN, digested with collagen and the presence of remaining fluorescent DCs analyzed (three independent experiments, **p=0,0034, *p=0,0445).
**E:** Migration of WT, Ii^{-/-} and CatS^{-/-} BM-DCs in uncoated transwells. 10⁵ cells were incubated with LPS (10µg/ml) in the upper well of the chamber, the lower well containing CCL19 and CCL21 (1µg/ml each, NC=no chemokines). Migrated cells were counted 3h later.
**F:** Migration of WT, Ii^{-/-} and CatS^{-/-} BM-DCs in collagen 1-coated transwells. 3.10⁵ cells were incubated with LPS (10µg/ml) in the upper well of the chamber, the lower well containing CCL19 and CCL21. Migrated cells were counted 24h later. **E, F:** the results are expressed in percentage and normalized to migration of WT DCs (mean+/-SD, three independent experiments, **p=0,001, *p=0,01).
**G:** Inhibition of DC migration by the CatS inhibitor LHVS (25nM, overnight). The experiment was performed as in F. The results are represented as the percentage of LHVS-treated DCs that migrated relative to untreated cells (mean+/-SD, three independent experiments, ***p=0.0005).
**H:** Ii depletion upon infection of day 4 BM-DCs with a lentivirus encoding an Ii shRNA. Infected BM-DCs were collected at day 10, lysed and analyzed by SDS-PAGE followed by immunoblotting using anti-Ii or an anti-Syk Ab.
**I:** Migration of BM-DCs depleted for Ii was analyzed in collagen-coated transwells as described in F. The results are expressed in percentage and normalized to migration of control WT DCs (mean+/- SD, two independent experiments, **p=0.005, *p=0.05).

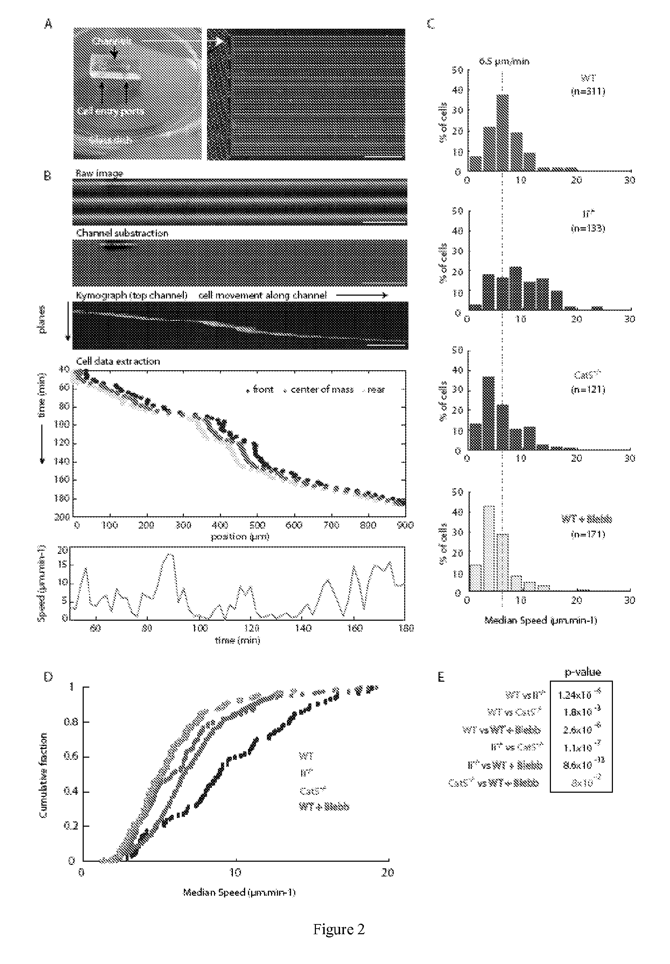

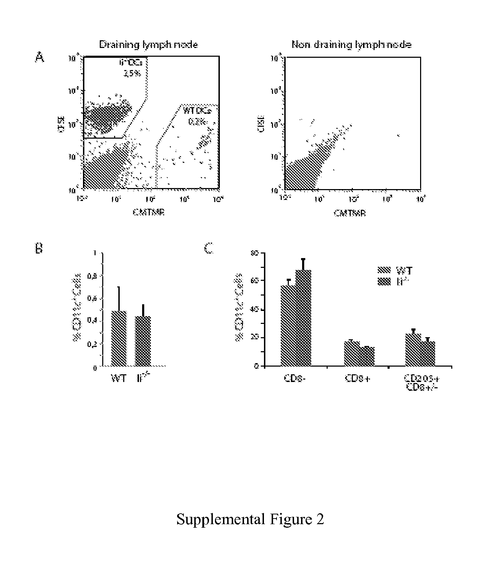

### Figure 2. Ii decreases the velocity of DCs migrating in confining micro-channels.

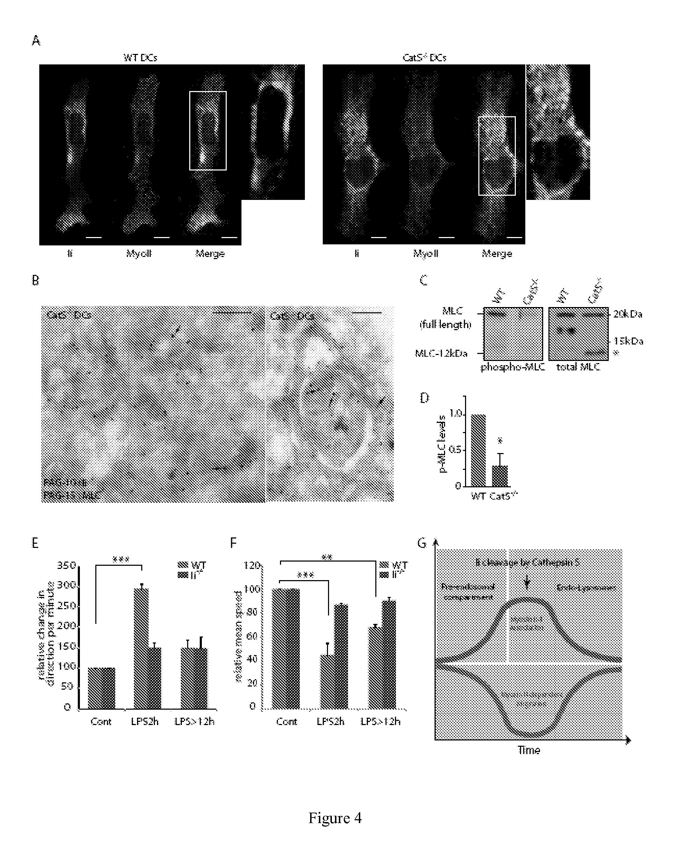

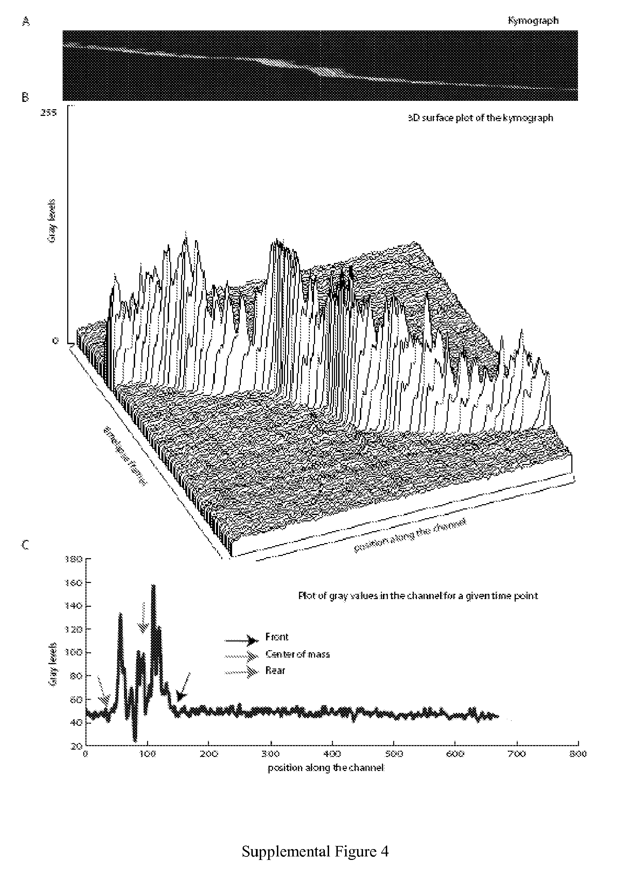

**A:** Experimental set-up: left, PDMS 4µm parallel fibronectin-coated channels on a glass-bottom dish. Right, 4x phase contrast image of the channels. Scale bar=200µm.
**B:** Data extraction process. Raw phase contrast images acquired with a 10x objective were processed as described in materials and methods to substract the channel contribution to the image (scale bar=50µm). The pictures were obtained from movie 1 (top channel). Upon channel substraction, kymographes were assembled from the movie, which contains in a very dense format all the necessary informations to analyze cell movement (scale bar=100µm). The positions of the front, rear and center of mass of the cell in each line of the kymograph were extracted (as illustrated in Sup.Fig.4) and used to measure instantaneous cell velocities. The speed shown on the bottom graph is the speed of the center of mass of the cell.
**C, D:** Distribution of median instantaneous speeds for WT, Ii^{-/-}, CatS^{-/-} DCs and WT DCs treated with 20µM blebbistatin. In D, speed distribution is represented in a cumulative graph to allow statistical analysis.
**E:** the p-values obtained from graph D using the Kolmogorov-Smirnov test indicate that all distributions are significantly different from each other with a 95% confidence interval, to the exception of CatS^{-/-} and blebbistatin-treated DCs, which display similar velocity distributions.

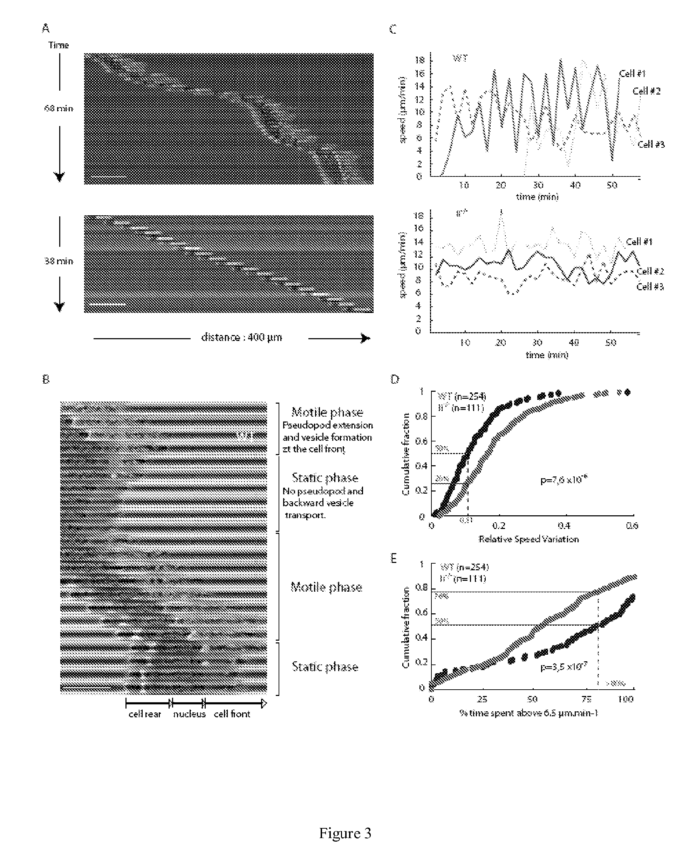

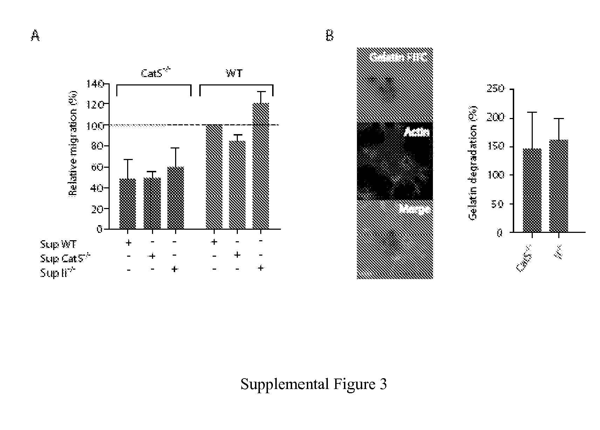

### Figure 3. Ii imposes a dis-continuous migration mode to DCs.

**A:** DCs migrating along a 4µm fibronectin-coated micro-channel, covering a distance of 400µm. 10x objective phase sequential images of temporal sequences were placed one below the other (vertical axis) after image cleaning. The interval between two images is 2 min. The two cells shown are representative of the behavior of WT (movie 1) and Ii^{-/-} (movie 2) DCs. Scale bar= 50µm.
**B:** Sequential high resolution (100x) phase contrast images of a WT cell (movie 1) migrating in a micro-channel. The channel part displayed is 88 microns long (vertical axis) and the time between the first and the last image is 14min (40s between each image, horizontal axis). The cell alternates high motility phases, during which pseudopods extend at the cell front leading to vesicle formation, with low motility phases during which these vesicles are transported backward. Note the contractile ring located at the cell rear. Scale bar= 20µm.
**C:** Representative examples of velocities displayed by individual WT, Ii^{-/-} and CatS^{-/-} DCs as a function of time. The data were extracted from movies 1 and 2. WT cell#1 and Ii^{-/-} cell#2 correspond to the DCs shown in A.
**D:** Cumulative distribution of the relative speed variations of WT and Ii^{-/-} DCs. The relative speed variation was calculated as the average of the local deviation from the local mean (2 points before and two points after each time point) divided by the global median velocity.
**E:** Cumulative distribution of the percentage of time spent by WT and Ii^{-/-} DCs migrating at speeds above 6.5)µm.min⁻¹.

### Figure 4. Variations in the levels of Ii in non-activated and LPS-activated DCs modify the localization and phosphorylation of Myosin II as well as DC motility.

**A:** Immunofluorescence analysis of MyoII HC and Ii sub-cellular localization in WT and CatS^{-/-} BM-DCs that were plated on line-shape fibronectin-coated micro-patterns (50x10µm) prior to fixation, permeabilization and staining. Confocal sections show that Myosin II staining is enriched in Ii-containing compartments that form in CatS^{-/-} DCs. Scale bar=2µm.
**B:** Cryoimmunoelectron microscopy images of CatS^{-/-} BM-derived DCs. The co-localization of Ii and Myosin II light chain (MLC) were analyzed using secondary Abs coupled to protein A-gold (PAG) of 15nm and 10nm of diameter respectively. The two panels show endo-lysosomal compartments containing both Ii and MLC in CatS^{-/-} DCs. Scale bar=200nm (left panel) and 500nm (right panel).
**C:** WT and CatS^{-/-} DCs were lysed and analyzed by SDS-PAGE and immunoblotting using anti-phospho-MLC (Threo18/Ser19) and anti-total MLC Abs sequentially. The *MLC-12KDa is not phosphorylated on Threo18/Ser19.
**D:** MLC phosphorylation immunoblots were quantified using the Image J software. The results were normalized to the expression levels of actin or tubulin and expressed relatively to MLC phosphorylation in WT DCs (three independent experiments, *p=0.01).
**E-F:** The impact of LPS on DC motility. Cells were pre-treated with LPS for 2h or >12h and their motility along micro-channels was analyzed by time-lapse imaging. Data were extracted as described in figure 2A. The frequency of changes per minute in the direction of cell migration was computed for individual cells as the number of times that they change direction relative to the total length of the run (panel E). Mean velocities were calculating by averaging the instantaneous velocity values exhibited by an individual cell during the whole run taking into account their directionality, such that if a cell goes back and forth its average speed is zero (panel F). All the values obtained from LPS-treated DCs are expressed as the percentage of the ones displayed by untreated cells from the same type (** = p <0.001, *** =p <0.0001; 100-200 cells per condition from two independent experiments).
**G:** Model explaining the regulation of DC migration by Myosin II, Ii and Cathepsin S.

### Figure S1. Phenotypes of WT, Ii-/- and CatS-/- Bone Marrow-derived DCs

**A:** Flow cytofluorometry analysis of day-12 GM-CSF-derived BM-DCs generated from WT, Ii-/- and CatS-/- mice and treated or not with LPS (10ug/ml, overnight). Cells were analyzed for surface expression of the DC marker CD11c, MHCII (I-Ab) and B7.2.
**B:** Flow cytofluorometry analysis of day-12 GM-CSF-derived bone marrow DCs generated from WT, Ii-/- and CatS-/- mice. Cells were analyzed for total expression of Ii using the IN-1 Ab. A, B: The histograms shown were obtained by gating on CD11c+ cells (>80% of the culture).
**C:** Immunoblot analysis of Ii expression in day-12 BM-derived DCs generated from WT, Ii-/- and CatS-/- mice. Cells extracts were separated on a 12% SDS-PAGE gel, transferred to a PVDF membrane and revealed with the anti-Ii IN-1 Ab.

### Figure S2. In vivo migration of WT and Ii-/- DCs

**A:** Flow cytofluorometry analysis of DC migration from the footpad to the popliteal LN. The experiment was performed as described in figure 1A. The percentage of Ii-/- DCs that have reached the popliteal LN 48h after footpad injection is increased, as compared to WT cells.
**B-C:** Quantification by flow cytofluorometry of DC sub-populations in LNs from WT and Ii-/- mice. A Single cell suspension obtained from cervical and/or inguinal LNs was stained with anti-CD11c, CD8 and CD205 Abs. Panel B shows the percentage of CD11c+ cells in total LNs. Panel B shows the percentage of the various DC subsets after gating on the CD11c+ population.

No significant difference is observed between WT and Ii-/- mice (three independent experiments).

### Figure S3. No soluble factors involved in regulation of DC migration by Ii

**A:** Supernatant transfer experiments in collagen I-coated transwells. Supernatants of overnight LPS-stimulated WT, Ii-/-, or CatS-/- BM-DCs were collected and added to CatS-/- or WT cells before transfer to the upper chamber of the transwell. 24h later, migrated cells were collected from the lower well, which contained a mixture of CCL19 and CCL21 chemokines, and counted. The results were expressed as percentages and normalized to migration of WT DCs incubated with WT supernatant (=100%, mean+/-SD, three independent experiments).
**B:** ECM degradation by WT, Ii-/- and CatS-/- DCs. Cells were plated on cross-linked gelatin-FITC for 6h, fixed and stained with red phalloïdin to label the actin cytoskeleton. A substrate level optical section shows the dark areas of gelatin-FITC degradation. These areas were quantified using the Metamorph software as described in materials and methods. Gelatin-FITC degradation by Ii-/- and CatS-/- DCs was expressed in percentages, relative to the degradation observed in WT DCs (=100%, mean+/-SD, three independent experiments).

### Figure S4. Process of data extraction from kymographs

**A.** Kymograph shown in figure 2B.
**B.** 3D surface plot obtained from the kymograph displayed in A by plotting the grey values of each line of the kymograph, which individually corresponds to one frame of the time-lapse movie. Thus, the movie of a cell migrating along a micro-channel, the kymograph assembled from it and the surface plot extracted from the kymograph, all contain the same information .
**C.** To extract cell position, a threshold is first set. A local followed by a global analyses are then applied to extract each cell track and identify the edges of the cell in each line (often, when long pseudopods extend forward, the extended parts are detected as an independent structure first and then reattributed to the cell on size criteria that are calculated on the whole kymograph). The center of mass is determined as the center of mass of intensities in the region between the front and the cell rear. This is meant to distinguish the cell body from the long thin extension of pseudopods at the cell front. All the measures given in the figures for speed correspond to the velocity of the cell center of mass.

### Figure S5. Myosin II inhibition impairs the migration of WT and Ii-/- DCs

**A.** BM-DCs were incubated in complete medium containing LPS (10g/ml) plus or minus the Myosin II inhibitor, Blebbistatin. Cells were immediatly transfered to the upper chamber of the transwell. 24h later, migrated cells were collected from the lower well, which contained a mixture of CCL19 and CCL21 chemokines, and counted. The results were expressed as percentages (mean+/-SD, three independent experiments, *** = p<0,0001, ** = p<0,001). NC= no chemokine
**B.** Inhibition of migration of WT and Ii-/- DCs by Blebbistatin (20M). The experiment was performed as in A. The results are represented as the percentage of Blebbistatin-treated DCs that migrated relative to untreated cells (mean+/-SD, three independent experiments).

### Figure S6. Co-localization between Myosin II and Ii in WT DCs

**A:** Immunofluorescence and confocal analysis of BM-DCs plated on line-shape micro-patterns (50x10mm) coated with green fluorescent fibronectin. Cells were incubated on patterns during 45min at 37°C prior to fixation, permeabilization and staining with an anti-LAMP-1 Ab.
**B:** Immunofluorescence analysis of MyoII HC and Ii sub-cellular localization in WT BM-DCs that were treated or not with LHVS and plated on line-shape fibronectin-coated micro-patterns (50x10m) prior to fixation, permeabilization and staining. 3-D projections of confocal stacks are shown. Myosin II staining is enriched in Ii-containing compartments that form in LHVS-treated WT DCs. Scale bar=2m
**C:** Cryoimmunoelectron microscopy analysis of WT BM-derived DCs. The sub-cellular distribution of Ii and Myosin II light chain (MLC) were analyzed using secondary Abs coupled to protein A-gold (PAG) of 15nm and 10nm of diameter respectively. Scale bar=500nm.

### Figure S7. Myosin II localization in WT and CatS-/- DCs

**A:** Immunoblot analysis of lysates obtained from extracts of WT DCs enriched for endosomal compartments. DCs were incubated with magnetic nano-particles, mechanically broken, centrifuged to remove nuclei and obtain the PNS (post-nuclear supernatant). The PNS was then separated on a magnet to generate the magnetic and non-magnetic fraction (MF and NMF), and lysed in NP-40-containing buffer. 15mg of lysates were separated on a 12% SDS-PAGE gel, transferred onto a PVDF membrane and incubated with the indicated Abs.
**B:** Analysis by SDS-PAGE and immunoblotting of endosomes semi-purified from WT and CatS-/- DCs. The results show an accumulation of Myosin II Heavy Chain (MyoHC) and full length MLC (20KDa) in endosomal membranes from CatS-/- DCs. A fragment of MLC (*MLC12kDa) was detected in CatS-/- DCs exclusively.
**C:** Amino-acid sequence of the mouse Myosin II regulatory light Chain. Serine 18 and threonine 19, which become phosphorylated when the protein is activated, are displayed in blue. The peptide identified by mass-spectrometry as contained in the 12kDa MLC fragment found in CatS-/- DCs is shown in red and underlined.
**D:** WT and Ii-/- DCs were treated overnight with the CatS inhibitor LHVS (25nM), lysed and analyzed by SDS-PAGE and immunoblotting using anti-MLC and anti-tubulin Abs. The *MLC-12KDa fragment is detected only in LHVS-treated WT DCs.

### Figure S8. Myosin II-Ii association in non-activated and activated DCs

**A:** WT BM-DCs were treated with LPS during different time periods and lysed. Myo II HC was immunoprecipitated and the pulled-down material was analyzed by immunoblotting with the IN-1 Ab, which recognizes the N-terminal portion of Ii. *corresponds to the Ig light-chain of the anti-Myo II HC Ab. LPS treatment transiently increases the association between Myosin II and Ii.
**B:** The experiment presented in panel A was quantified using the Image J software. The graph shows the changes in the amounts of pulled-down Ii relative to the amounts of immunoprecipitated Myo II HC at each time-point after LPS treatment.
**C:** WT DC pre-treated with LPS for 2h migrating along a 4m fibronectin-coated micro-channel. 10x objective phase sequential images of temporal sequences were placed one below the other (vertical axis) after image cleaning. The interval between two images is 2min. Scale bar= 50m.
**D:** The impact of LPS on DC motility. WT and Ii-/- DCs were pre-treated with LPS for 2h or >12h and their motility along micro-channels was analyzed by time-lapse imaging. Data were extracted as described in figure 2A and the median instantaneous velocities of individual cells were computed. All the values obtained from LPS-treated DCs are expressed as the percentage of the ones displayed by untreated cells from the same type (** = p <0.001, *** = p <0.0001; 100-300 cells per condition from two independent experiments).

### Figure S9. Ii decreases the velocity of BCR-stimulated B lymphocytes

**A :** Freshly purified resting mature IgM+/IgD+ B cells were incubated with BCR multivalent ligands and analyzed by time-lapse microscopy (one image per minute during 2h). Mean velocities were calculated by tracking each cell movement using the Metamorph software. About 50 cells were tracked per condition and per experiment (*** = p <0.0001, two independent experiments).

***Figure S10*** : Schematic assembly of the chamber. PDMS piece, with embedded microchannels and holes for the inlet and outlet ports, is sealed by plasma activation to a glass coverslip.

### DETAILED DESCRIPTION OF THE INVENTION

### MODULATION OF DENDR TIC CELL MIGRATION

The inventors identified the MHCII-associated Invariant Chan (also called CD74 or Li) as a key factor for regulating the motility and the migration of cells, in particular dendritic cells (DCs). The inventors showed that a decrease of CD74 increases the dendritic cell migration and, alternatively an increase of CD74 decreases the DCs migration. The polynucleotide and amino acid sequences of CD74 are well-known in the art. Some reference sequences are Genbank Accession Nos NM_010545.3 and NP_034675.1, respectively. The reference entry for human CD74 in the transcriptome database UniGene is Hs.436568.

Accordingly, the present invention concerns a CD74 modulator agent for regulating the dendritic cell migration; the use of a CD74 modulator agent for the preparation of a medicament for regulating the dendritic cell migration; and a method for regulating the dendritic cell migration in a subject comprising administering a therapeutically efficient amount of a CD74 modulator agent to the subject, thereby regulating the dendritic cell migration.

In a particular embodiment, the present invention concerns a CD74 inhibitor agent for increasing the dendritic cell migration; the use of a CD74 inhibitor agent for the preparation of a medicament for increasing the dendritic cell migration; and a method for increasing the dendritic cell migration in a subject comprising administering a therapeutically efficient amount of a CD74 inhibitor agent to the subject, thereby increasing the dendritic cell migration.

In an alternative embodiment, the present invention concerns a CD74 activator agent for decreasing the dendritic cell migration; the use of a CD74 activator agent for the preparation of a medicament for decreasing the dendritic cell migration; and a method for decreasing the dendritic cell migration in a subject comprising administering a therapeutically efficient amount of a CD74 activator agent to the subject, thereby decreasing the dendritic cell migration.

### CD74 inhibitors

By CD74 inhibitors is intended any molecule allowing to decrease the expression or the activity of CD74.

Thus, the inhibitor can induce the suppression or the reduction of the transmission of extracellular signals into the cell through CD74 receptor. The activity of CD74 receptor can be easily assayed by any method known in the art. A first assay can be the determination of the ability of the inhibitor to bind the CD74 receptor. A second assay can be the determination of the ability of the inhibitor to compete with a ligand of the CD74 receptor for the binding of this receptor or of this ligand. A third assay can be the determination of the ability of the inhibitor to decrease the CD74 expression level. These different methods are described below in this document and can be combined.

The inhibition can be due to the binding of a molecule on the extracellular domain of the receptor. In this case, the inhibitor can be an antagonist which binds to the ligand binding domain or another domain of the receptor, or a molecule which modifies the activity of the receptor by steric hindrance or modification. This inhibitor can be, for instance, a small molecule, an aptamer or an antibody directed against the extracellular domain of the receptor. The inhibitory activity can be determined through a binding assay, a competitive binding assay or a phosphorylation assay.

The inhibition can also be due to the reduction or suppression of the expression of the gene coding for the receptor, for example by using specific RNAi, antisense or ribozyme, which induces a decrease of the number of receptors at the cell surface and thus a reduction of the extracellular signal transmission. The inhibitory activity can be assayed through the measure of the expression level of CD74, at the protein level or RNA level.

The use of baits which bind ligands of the CD74 receptor can also induce reduction or suppression of the activity of this receptor by competition for these ligands. Indeed, these baits trap ligands of CD74 and, consequently, decrease the concentration of available ligands for CD74 activation. These baits can be disposed in the membrane such as dominant negative receptors or in the extracellular fluid such as soluble receptor. The inhibitory activity can be determined through a competition assay in order to determine the decrease of binding between the functional CD74 receptor and its ligand.

In preferred embodiments of the present invention, the CD74 inhibitor is preferably selected from the group consisting of small molecule inhibiting MIF (Macrophage migration inhibitory factor), an antibody directed against CD74, in particular against the extracellular domain of CD74, a nucleic acid molecule interfering specifically with CD74 expression, and a CD74 soluble bait.

As used herein, the term "small molecule inhibiting MIF" refers to small molecule that can be an organic or inorganic compound, usually less than 1000 daltons, with the ability to inhibit or reduce the activity of MIF. This small molecule can be derived from any known organism (including, but not limited to, animals, plants, bacteria, fungi and viruses) or from a library of synthetic molecules. Small molecules inhibiting MIF can be identify with the method further describe in this document.

In a particular embodiment, this molecule is selected from the group consisting of (S,R)-3-(4-hydrophenyl)-4,5-dihydro-5-isoazole acetic acid methyl ester (ISO-1) and MIF inhibitors disclosed in US2007/0219189, WO2007/070961, US2003/0195194 (the disclosure of which is incorporated herein by reference).

The inhibitor is a anti-CD74 antibody having an antagonist activity. As used herein, the term "antibody" is intended to refer broadly to any immunologic binding agent such as IgG, IgM, IgA, IgD and IgE, and humanized or chimeric antibody. In certain embodiments, IgG and/or IgM are preferred because they are the most common antibodies in the physiological situation and they are most easily manufactured. The term "antibody" is used to refer to any antibody-like molecule that has an antigen binding region, and includes antibody fragments such as Fab', Fab, F(ab') 2, single domain antibodies (DABs), Fv, scFv (single chain Fv), and the like. The techniques for preparing and using various antibody-based constructs and fragments are well known in the art. Means for preparing and characterizing antibodies are also well known in the art (See, e.g., Harlow and Lane, 1988).

A "humanized" antibody is an antibody in which the constant and variable framework region of one or more human immunoglobulins is fused with the binding region, e.g. the CDR, of an animal immunoglobulin. "Humanized" antibodies contemplated in the present invention are chimeric antibodies from mouse, rat, or other species, bearing human constant and/or variable region domains, bispecific antibodies, recombinant and engineered antibodies and fragments thereof. Such humanized antibodies are designed to maintain the binding specificity of the non-human antibody from which the binding regions are derived, but to avoid an immune reaction against the non-human antibody.

A "chimeric" antibody is an antibody molecule in which (a) the constant region, or a portion thereof, is altered, replaced or exchanged so that the antigen binding site (variable region) is linked to a constant region of a different or altered class, effector function and/or species, or an entirely different molecule which confers new properties to the chimeric antibody, e.g., an enzyme, toxin, hormone, growth factor, drug, etc.; or (b) the variable region, or a portion thereof, is altered, replaced or exchanged with a variable region having a different or altered antigen specificity.

Particularly, the term "antibody against the extracellular domain of CD74" designates an antibody as described above which is able to bind to the extracellular domain of the CD74 receptor and to block or reduce its activity. This inhibition can be due to steric hindrance or modification which prevents ligand binding.

In a preferred embodiment, the antibody directed against the extracellular domain of CD74 is CD74(C-16) : sc-5438 (Santa-Cruz biotechnology). Such antibodies can be obtained by any method known in the art and for instance such antibodies are disclosed in WO2004/110390.

As used herein, the term "CD74 soluble bait" designates an extracellular molecule which is able to bind to a CD74 ligand. This soluble bait can be constituted of any peptide which has the ability to bind a ligand of the CD74 receptor.

In one embodiment, the CD74 soluble bait is a recombinant CD74 receptor constituted of the extracellular domain of the receptor. In a preferred embodiment, the CD74 soluble bait is the entire extracellular domain of CD74 receptor.

The inhibitors of CD74 of the invention may also be nucleic acid molecules. The terme "nucleic acid molecule" includes, but is not limited to, RNAi, antisense and ribozyme molecules.

In the present invention, a "nucleic acid molecule specifically interfering with CD74 expression" is a nucleic acid molecule which is able to reduce or to suppress the expression of gene coding for CD74 receptor, in a specific way.

The term "RNAi" or "interfering RNA" means any RNA which is capable of down-regulating the expression of the targeted protein. It encompasses small interfering RNA (siRNA), double-stranded RNA (dsRNA), single-stranded RNA (ssRNA), micro-RNA (miRNA), and short hairpin RNA (shRNA) molecules. RNA interference, designate a phenomenon by which dsRNA specifically suppresses expression of a target gene at post-translational level. In normal conditions, RNA interference is initiated by double-stranded RNA molecules (dsRNA) of several thousands of base pair length. In vivo, dsRNA introduced into a cell is cleaved into a mixture of short dsRNA molecules called siRNA. The enzyme that catalyzes the cleavage, Dicer, is an endo-RNase that contains RNase III domains (Bernstein, Caudy et al. 2001). In mammalian cells, the siRNAs produced by Dicer are 21-23 bp in length, with a 19 or 20 nucleotides duplex sequence, two-nucleotide 3' overhangs and 5'-triphosphate extremities (Zamore, Tuschl et al. 2000; Elbashir, Lendeckel et al. 2001; Elbashir, Martinez et al. 2001).

A number of patents and patent applications have described, in general terms, the use of siRNA molecules to inhibit gene expression, for example, WO 99/32619, US 20040053876, US 20040102408 and WO 2004/007718.

siRNA are usually designed against a region 50-100 nucleotides downstream the translation initiator codon, whereas 5'UTR (untranslated region) and 3'UTR are usually avoided. The chosen siRNA target sequence should be subjected to a BLAST search against EST database to ensure that the only desired gene is targeted. Various products are commercially available to aid in the preparation and use of siRNA.

In a preferred embodiment, the RNAi molecule is a siRNA of at least about 15-50 nucleotides in length, preferably about 20-30 base nucleotides, preferably about 20-25 nucleotides in length.

In a particular embodiment, the siRNA molecule comprises the sequences discloses in the experimental section.

RNAi can comprise naturally occuring RNA, synthetic RNA, or recombinantly produced RNA, as well as altered RNA that differs from naturally-occurring RNA by the addition, deletion, substitution and/or alteration of one or more nucleotides. Such alterations can include addition of non-nucleotide material, such as to the end of the molecule or to one or more internal nucleotides of the RNAi, including modifications that make the RNAi resistant to nuclease digestion.

RNAi may be administered in free (naked) form or by the use of delivery systems that enhance stability and/or targeting, e.g., liposomes, or incorporated into other vehicles, such as hydrogels, cyclodextrins, biodegradable nanocapsules, bioadhesive microspheres, or proteinaceous vectors (WO 00/53722), or in combination with a cationic peptide (US 2007275923). They may also be administered in the form of their precursors or encoding DNAs.

Antisense nucleic acid can also be used to down-regulate the expression of the CD74 receptor. The antisense nucleic acid can be complementary to all or part of a sense nucleic acid encoding a CD74 receptor polypeptide e.g., complementary to the coding strand of a double-stranded cDNA molecule or complementary to an mRNA sequence, and it thought to interfere with the translation of the target mRNA

In a preferred embodiment, the antisense nucleic acid is a RNA molecule complementary to a target mRNA encoding a CD74 receptor polypeptide.

An antisense nucleic acid can be, for example, about 5, 10, 15, 20, 25, 30, 35, 40, 45 or 50 nucleotides in length. Particularly, antisense RNA molecules are usually 18-50 nucleotides in length.

An antisense nucleic acid for use in the method of the invention can be constructed using chemical synthesis and enzymatic ligation reactions using procedures known in the art. Particularly, antisense RNA can be chemically synthesized, produced by *in vitro* transcription from linear (e.g. PCR products) or circular templates (e.g., viral or non-viral vectors), or produced by *in vivo* transcription from viral or non-viral vectors.

Antisense nucleic acid may be modified to have enhanced stability, nuclease resistance, target specificity and improved pharmacological properties. For example, antisense nucleic acid may include modified nucleotides designed to increase the physical stability of the duplex formed between the antisense and sense nucleic acids, e.g., phosphorothioate derivatives and acridine substituted nucleotides.

Ribozyme molecules can also be used to decrease levels of functional CD74. Ribozymes are catalytic RNA molecules with ribonuclease activity which are capable of cleaving a single-stranded nucleic acid, such as an mRNA, to which they have a complementary region. Thus, ribozymes can be used to catalytically cleave mRNA transcripts to thereby inhibit translation of the protein encoded by the mRNA. Ribozyme molecules specific for functional CD74 can be designed, produced, and administered by methods commonly known to the art (see e.g., Fanning and Symonds, 2006, reviewing therapeutic use of hammerhead ribozymes and small hairpin RNA).

### CD74 activators

By CD74 activators is intended any molecule allowing to increase the expression or the activity of CD74.

Thus, the activator can induce the increase of the transmission of extracellular signals into the cell through CD74 receptor. The activity of CD74 receptor can be easily assayed by any method known in the art. A first assay can be the determination of the ability of the activator to bind the CD74 receptor. A second assay can be the determination of the ability of the activator to agonize the CD74 receptor. A third assay can be the determination of the ability of the activator to increase the CD74 expression level. These different methods are described below in this document and can be combined.

A first possible CD74 activator is a recombinant MIF or a MIF agonist. MIF agonists include, but are not limited to, those disclosed in WO/2006/116688, WO/2007/044622,

A second possible CD74 activator is an inhibitor of Cathepsin S (CatS). Known CatS inhibitors useful in the present invention include, but are not limited to, dipeptide nitriles described in WO99/24460, α-amino fluoro ketones described in US 4,518,528, peptides with fluoride free leaving groups as described in US 5,374,623, compounds with heterocyclic leaving groups as described in US 5,486,623 and compounds containing alkyl sulfonyls such as described in US 6,030,946. Additional references disclosing CatS inhibitors include : WO00/49007, WO00/49008, WO97/40066, WO96/40737, WO01/19816, WO00/55125, US7,326,716 and WO00/51998. (the disclosure of all of which are incorporated herein by reference). Examples of selective CatS inhibitor are LHVS (Morpholinurea-leucine-homophenylalanine-vinylsulfone-phenyl (from Arris Pharmaceutical) and 1-[3-[4-(6-Chloro-2,3-dihydro-3-methyl-2-oxo-1H-benzimidazol-1-yl)-1-piperidinyl]propyl]-4,5,6,7-tetrahydro-5-(methylsulfonyl)-3-[4-(trifluoromethyl)phenyl]-1H-pyrazolo[4,3-c]pyridine. (JNJ 10329670).

A thrid possible possible CD74 activator is an agonist anti-CD74 antibody.

Thus, the activator can induce the increase of the transmission of extracellular signals into the cell through CD74 receptor. The activity of CD74 receptor can be easily assayed by any method known in the art. A first assay can be the determination of the ability of the activator to bind the CD74 receptor. A second assay can be the determination of the ability of the activator to agonize the CD74 receptor. A third assay can be the determination of the ability of the activator to increase the CD74 expression level. These different methods are described below in this document and can be combined.

The pharmaceutical composition comprising the CD74 modulator agent is formulated in accordance with standard pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art.

Possible pharmaceutical compositions include those suitable for oral, rectal, intravesial, topical (including transdermal, buccal and sublingual), or parenteral (including subcutaneous, intramuscular, intravenous and intradermal) administration. For these formulations, conventional excipient can be used according to techniques well known by those skilled in the art.

The compositions for parenteral administration are generally physiologically compatible sterile solutions or suspensions which can optionally be prepared immediately before use from solid or lyophilized form. Adjuvants such as a local anesthetic, preservative and buffering agents can be dissolved in the vehicle and a surfactant or wetting agent can be included in the composition to facilitate uniform distribution of the active ingredient.

For oral administration, the composition can be formulated into conventional oral dosage forms such as tablets, capsules, powders, granules and liquid preparations such as syrups, elixirs, and concentrated drops. Non toxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials are also necessary. For example, starch, gelatine, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants.

For transdermal administration, the composition can be formulated into ointment, cream or gel form and appropriate penetrants or detergents could be used to facilitate permeation, such as dimethyl sulfoxide, dimethyl acetamide and dimethylformamide.

For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

Pharmaceutical compositions according to the invention may be formulated to release the active drug substantially immediately upon administration or at any predetermined time or time period after administration.

The present invention can be useful in the treatment or prevention of auto-immune diseases, cancers, inflammatory diseases and viral infections.

The present invention can be useful in the treatment or prevention of immune system pathologies such as auto-immune diseases and inflammatory diseases (such as atherosclerosis) or disorders. Indeed, in such diseases or disorders, the immune cells are accumulated. Use of CD74 inhibitor agent allows the stimulation of migration, thereby preventing their cell accumulation on the site of disease.

Similarly, the present invention can also be useful in anti-tumor vaccine. Indeed, use of CD74 inhibitor agent allows the stimulation of migration of DC used in therapeutic vaccine, thereby facilitating the ability to reach ganglions for stimulating the immune response. Today, the DCs migration remains the limiting step of the anti-tumor vaccine protocols. Accordingly, the present invention concerns a composition comprising a anti-tumor vaccine and a CD74 inhibitor agent, a product comprising a anti-tumor vaccine and a CD74 inhibitor agent for a simultaneous or sequential use for treating cancer. It also concerns a method for increasing the efficiency of a anti-tumor vaccine in a subject comprising administering to the subject an efficient amount of CD74 inhibitor agent. It further concerns a CD74 inhibitor agent for increasing the efficiency of a anti-tumor vaccine. Anti-tumor vaccines are for instance disclosed in the following documents (the disclosure of which is incorporated herein by reference) : WO2008108679, WO2007126163, US2007243197, WO2006059785, WO02074338.

Alternatively, the CD74 activator can be used to increase the expression of CD74 in tumor cells, thereby decreasing the ability of a tumor cell to leave the primary tumor and to invade healthy tissues. Indeed, some tumor cells are known to express CD74 (e.g., melanoma).

### DEVICES AND METHODS FOR DETERMINING CELL MOTILITY

In the present experimental section, the inventors used micro-fabricated channels to investigate the impact of the Ii-Myosin II complex on DC migration (4 µm diameter). Microchannels offer several advantages for the study of DC motility: they confine the cells, mimicking the constrained microenvironment of peripheral tissue and lymphoid organ interstitial spaces, and they impose a directional migration of cells in the absence of chemokine gradient, allowing automatic analysis of cellular parameters (cell velocity, time of pauses, persistence) on large cell numbers. The use of micro-channels permitted the inventors to show that li negatively regulates DC motility by inducing transient slowing down phases during cell motion, an effect that depends on Myosin II activity.

Accordingly, a new model for cell migration, specific to confined environment is proposed. This model allows testing the properties of cell migration (distribution of velocity, persistence, pausing times, change in direction).

The design of the chamber containing the micro-channels allows cells to enter the channels by themselves after loading in an entry plug, or to force the cells inside the channels if needed (for cells which would not be motile outside of the channels, or for surface treatment preventing cell adhesion and thus motility outside of the channels). Multiple channels can be ran in parallel (e.g., around 50 channels per chamber), allowing an easy recording of many cells at low magnification. The bottom glass coverslip closing the channel is compatible with any type of high-resolution fluorescence microscopy (including TIRF, an important technique to follow actin dynamics close to the glass surface and thus understand how cells apply a force on the substrate as they move).

Accordingly, the device for determining or studying the motility capacity of a cell comprises at least one embedded micro-channel with holes for the inlet and outlet ports, said micro-channel having diameter smaller than the cell diameter. In particular, the micro-channels are embedded in a it comprises a plate defining a surface supporting the embedded micro-channel. The plate is a support convenient for confocal, optical and/or fluorescence microscopies. In the more preferred embodiment, the plate is glass, preferably silanised glass. For example, a convenient plate according to the present invention is a coverslip or a slide.

Each micro-channel can have any appropriate section form. For instance, the section form can be spherical, half-spherical, squared or rectangular. The diameter of the micro-channel is chosen so that the tested cell is constrained. More particularly, the diameter can be smaller than the diameter of the studied cell on a support without any constrain. For instance, the diameter of the micro-channel is between 1-20 µm, preferably between 1-10 µm. For DCs, the diameter can be around 4-5 µm. The length of the micro-channel is not an important parameter. Preferable, the length is longer than 100 µm. For instance, the length is between 100 µm and few mm, preferably between 1-10 mm, more preferably between 5-10 mm.

Preferably, the micro-channel inner surface is coated by molecules that promote cell attachment. These molecules are well known to those of ordinary skilled in the art and comprise antigens, antibodies, cell adhesion molecules, extracellular matrix molecules such as laminin, fibronectin, synthetic peptides, carbihydrates and the like. Preferably, said adhesive patterns comprise extracellular matrix molecules, more preferably fibronectin.

Standard methods are well known by those skilled in the art to prepare the device according to the present invention. The piece with embedded micro-channel can be poly(dimethylsiloxane) (PDMS) or an other siloxane-based polymer.

The present invention also concerns the use of a device according to the present invention for determining or studying the motility capacity of a cell. The cell is an eukaryotic cell, preferably a cell having a motility capacity. Preferably, the cell is a mammalian cell, more preferably a human cell. The cell can be a APC or DC, a cancer cell (e.g., an invasive cancer cell), or a fibroblast. The present invention also concerns the use of such a device for screening a molecule able to change the motility capacity of a cell.

The present invention concerns a method for determining or studying the motility capacity of a cell, comprising introducing the cell into the inlet port of a device of the present invention and measuring the move, the velocity of the cell into the micro-channel. The cell position can be measured by any convenient method, for instance confocal, optical and/or fluorescence microscopies.

The cell can be contacted with a test molecule in order to determine the effect of the molecule on the capacity of the cell to move. The test molecule may be of various origin, nature and composition. It may be any organic or inorganic substance, such as a lipid, peptide, polypeptide, nucleic acid, small molecule, etc., in isolated or in mixture with other substances. For instance, the test molecule can be an antibody, an antisense oligonucleotide, or an RNAi. The compounds may be all or part of a combinatorial library of products, for instance.

Further aspects and advantages of the present invention will be disclosed in the following experimental section, which should be regarded as illustrative and not limiting the scope of the present application.

### EXAMPLES

The sampling of peripheral tissues is an essential function of dendritic cells (DCs) that is achieved by coupling efficient antigen uptake and processing to high motility. In the present invention, the inventors show that the MHCII-associated Invariant Chain (Ii or CD74), a known regulator of antigen processing, negatively regulates DC motility and *in vivo* migration. By using micro-fabricated channels that mimic the confined environment of peripheral tissues, the inventors demonstrate that whereas wild-type DCs alternate high and low motility phases, Ii-deficient cells move in a uniform fast manner, indicating that Ii acts as a brake during DC locomotion. The present results further suggest that this peculiar property of Ii results from its association to the actin-based motor, Myosin II. Accordingly the inventors propose that the use of common mechanisms to regulate antigen processing and cell motility enables DCs to coordinate these two functions in time and space.

To investigate whether Ii regulates DC migration, the inventors compared the *in vivo* migratory properties of wild-type (WT) and Ii-deficient DCs (see Sup.Fig. 1A for DC phenotype). Bone marrow-derived DCs were loaded with fluorescent dyes (in the presence of LPS to induce CCR7 expression), transferred into the footpad of C57/BL6 mice, and their arrival to the draining lymph node (DLN) was monitored 48h after transfer. Remarkably, increased numbers of Ii-deficient DCs as compared to WT cells were found in the DLN of recipient mice (Fig.1A and Sup.Fig.2A). FITC-painting experiments of the ear gave rise to a similar observation: 24h after painting, enhanced numbers of endogenous DCs had reached the DLN in Ii^{-/-} mice as compared to their WT counterpart (Fig.1B). This difference did not result from passive diffusion of FITC since Ii^{+/+} and Ii^{-/-} mice displayed similar numbers of DCs in LNs at the steady state (Sup.Fig.2B). Hence, the absence of Ii facilitates the accumulation in LNs of activated DCs that migrate from the periphery.

The inventors next analyzed the migratory capacity of DCs from Cathepsin S (CatS)-deficient mice that display enhanced Ii protein levels (Sup.Fig.1A, B and C) (*10-12*). CatS cleaves Ii once MHCII-Ii complexes have reached endo-lysosomes, an event that is required for removal of Ii cytosolic tail, loading of antigenic peptide onto MHCII and transport of MHCII-peptide complexes to the cell surface (*10, 11*). In the absence of CatS, MHCII-Ii complexes accumulate in endo-lysosomes and, to a minor extent, at the plasma membrane (*12*). CatS^{-/-} DCs transferred into the footpad of C57/BL6 mice migrated less efficiently from the periphery to LNs as compared to WT DCs (Fig.2C). Analysis of the number of DCs in the footpad revealed increased numbers of CatS^{-/-} than WT cells, suggesting that CatS^{-/-} DCs remained in the footpad rather than homed to another tissue (Fig.2D). The inventors conclude that enhanced levels of Ii in CatS^{-/-} DCs correlate with reduced *in vivo* migration.

To investigate the mechanisms underlying these observations, the inventors compared the ability of WT, Ii^{-/-} and CatS^{-/-} DCs to migrate toward a CCL19/CCL21 gradient in transwells. The data obtained when using collagen I-coated transwells were consistent with our *in vivo* observations: whereas Ii-deficient cells migrated more efficiently than WT DCs, CatS^{-/-} cells showed impaired migration (Fig.1F). Migration of WT DCs was also enhanced when Ii was knocked-down using an Ii-shRNA encoded in a lentivirus (Fig.1I). Importantly, negative regulation of DC migration by Ii was not observed when using uncoated transwells (Fig.1E). This result indicates that Ii does not influence the ability of DCs to respond to CCL19/CCL21 chemokines but rather modifies their ability to move across a dense protein matrix. Transfer of supernatants between WT, Ii^{-/-} and CatS^{-/-} DCs had no effect on cell migration (Sup.Fig.3A). Furthermore, the status of Ii did not modify the capacity of DCs to degrade gelatin-FITC (Sup.Fig.3B). Both these findings suggest that the negative effect of Ii on DC migration in collagen-coated transwells does not involve the secretion of soluble factors.

The specific CatS inhibitor LHVS significantly reduced the migration of WT DCs (Fig.1G) (*13*). However, incubation of Ii-deficient DCs with LHVS did not compromise their migration (Fig.1G). This result indicates that the decreased migratory capacity of DCs whose CatS activity is impaired indeed resulted from Ii accumulation, and not from an Ii-independent action of the protease. This observation was further strengthened by showing that shRNA-depletion of Ii in CatS-deficient DCs restored their ability to migrate in collagen-coated transwells (Fig.1H and I). All together, our findings show that Ii negatively regulates the capacity of DCs to move across a protein matrix *in vitro* and *in vivo.*

To unravel the mechanisms by which Ii regulates DC migration, the inventors analyzed DC motility by time-lapse imaging using micro-fabricated 4µm-diameter channels (*14*) (Fig.2A). Two reasons motivated this choice. First, micro-channels enable cell confinement during motion and therefore mimic the microenvironment encountered by DCs in the constrained interstitial spaces of peripheral tissues and lymphoid organs (*15*). The physiological meaning of this specific point is highlighted by a recent study showing that DC motility on 2-D surfaces depends on Integrins and does not reflect *in vivo* migration, which occurs independently of these adhesion molecules (*6*). Second, micro-channels impose a directional migration to cells, which facilitates the extraction from important cell numbers of accurate measurable parameters, for example instantaneous velocities, what cannot be achieved in systems such as 3-D artificial collagen matrices. DCs entered and moved spontaneously and bi-directionally along micro-channels, with their cell body being constrained during motion (Fig.2B). Time-lapse movies obtained with WT, Ii^{-/-} and CatS^{-/-} DCs were analyzed by drawing kymographs and extracting cell positioning and instantaneous velocity data for individual migrating cells by using a homemade computer program (see materials and methods, Fig.2B and Sup.Fig.4). The distributions of median instantaneous velocities measured for each DC type are shown in figures 2C and 2D. Non-activated WT DCs displayed an average median instantaneous velocity of 6.5µm.min⁻¹ (Fig.2C). These values are compatible with the ones recently reported for BM-DC migrating in 3-D collagen matrices or in the mouse skin (*6*), validating micro-channels as an adequate tool to study DC motility. Strikingly, 71% of Ii^{-/-} DCs migrated at velocities above 6.5µm.min⁻¹, some of them reaching speeds 2 to 3-fold higher (Fig.2C, D, *p*=1.2x10⁻⁶). On the contrary, the velocities of 76% of CatS^{-/-} DCs fell below 6.5µm.min⁻¹ (Fig.2C, D, *p*=1.8x10⁻³). Hence, Ii levels regulate the speed of DCs that migrate in a confined microenvironment, Ii-deficiency favoring fast DC migration.

While imaging WT DCs migrating along micro-channels the inventors noticed that most of the cells underwent important speed fluctuations during motion, alternating high and low motility phases (Fig.3A). This irregular DC motion was even more remarkable when imaging cells at higher magnification, two phases being observed: a motile phase during which pseudopods were extended at the cell front, leading to the formation of big vesicles (most likely macropinosomes), followed by a rather static phase during which these vesicles were transported backwards (Fig.3B). Noticeably, Ii^{-/-} DCs showed a more regular, continuous movement, slow motility phases being rarely observed (Fig.3A). Even when comparing WT and Ii^{-/-} DCs that displayed a similar median velocity, the speed fluctuations undergone by cells during migration were reduced in the absence of Ii (Fig.3C). These results suggested that Ii regulates DC migration by inducing slowing-down phases during motion. Accordingly, when measuring the local speed variation of individual migrating DCs, the inventors obtained values significantly lower for Ii-deficient cells as compared to their WT counterpart, verifying on a large cell number that Ii^{-/-} DCs undergo less velocity variations during motion (Fig.3D, *p*=7.6x10⁻⁶). Indeed, half of Ii^{-/-} DCs displayed a speed variation value below 0.11, whereas this concerned only 26% of WT cells (Fig.3D). Furthermore, the inventors found that while 50% of Ii^{-/-} DCs spent most of their time (>80%) migrating faster than 6,5µm.min⁻¹ (the WT median speed), only 24% of WT DCs did so (Fig.3E). These results indicate that Ii-deficiency favors sustained DC migration at high speed. Thus, the inventors conclude that Ii reduces DC migration by transiently slowing-down DC velocity.

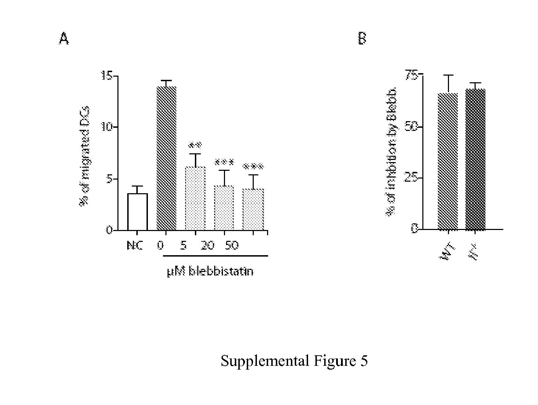

Having established that Ii modulates the speed of migrating DCs, the inventors next aimed at addressing the molecular mechanisms underlying such regulation. Using the Myosin II inhibitor, Blebbistatin, it was recently shown that this motor protein is required for DCs to migrate in collagen matrices as well as *in vivo* (*6*). Accordingly, the inventors found that treatment of DCs with Blebbistatin significantly impaired their migration in collagen-coated transwells, even when low Blebbistatin concentrations were used (<50µM, Sup. Fig.5A, Sup.movie 5). Migration of Ii-deficient DCs in collagen-coated transwells was also inhibited by Blebbistatin treatment (Sup. Fig.5B). This result indicates that as WT DCs, Ii^{-/-} DCs rely on the activity of Myosin II for migration. In micro-channels migration experiments, the inventors observed that the speed of WT DCs was significantly decreased when compromising Myosin II activity, 76% of the cells migrating at velocities below 6.5µm.min⁻¹ (WT median velocity, Fig.2C, 2D and 2E, *p-value*=2.6x10⁻⁷). Interestingly, Blebbistatin-treated DCs showed a velocity distribution similar to the one of CatS^{-/-} cells (Fig.2C and 2D) and statistical analysis showed no significant difference between both DC types (Fig.2E, *p-value*=8x10⁻²). This observation suggested that Myosin II activity might be compromised in CatS^{-/-} DCs. In view of the ability of Myosin II to interact with MHCII-Ii complexes in DCs (*7*), the inventors thus hypothesized that Ii retention in CatS^{-/-} DCs re-localizes Myosin II at the level of endosomal membranes, thereby affecting its activity.

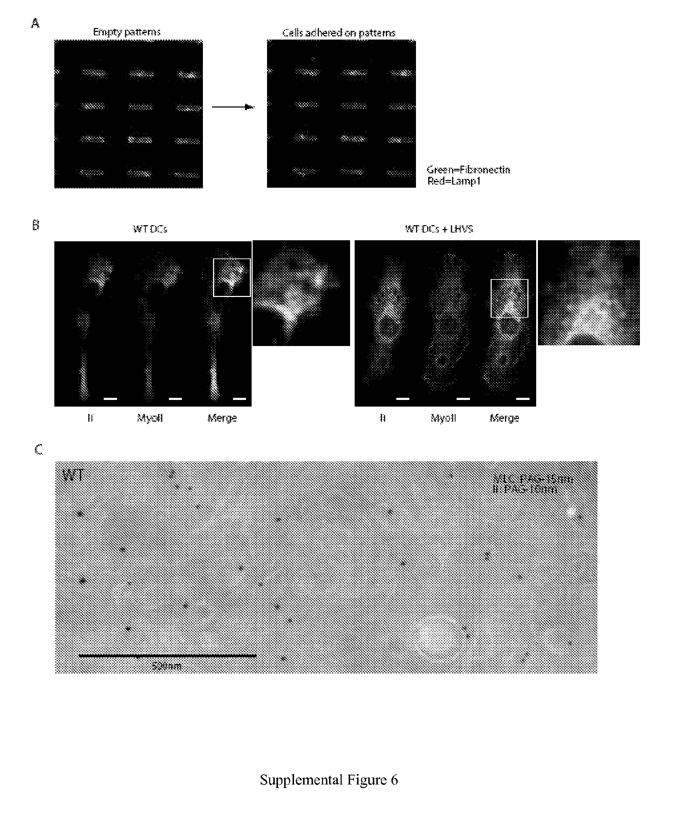

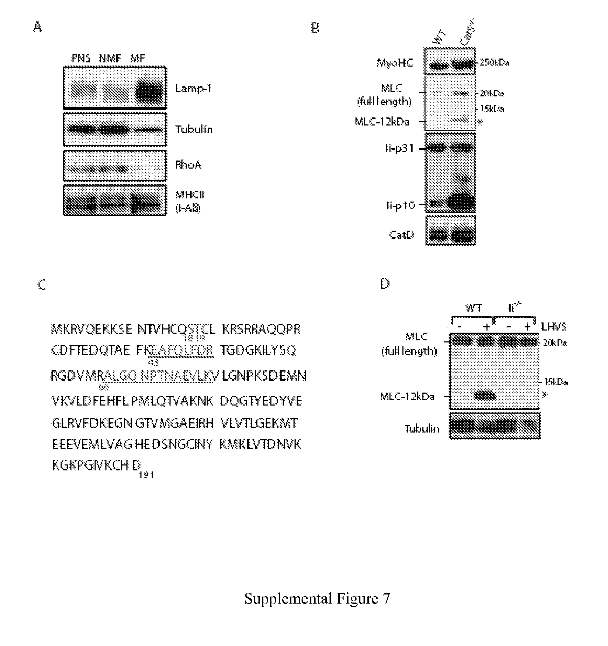

To address this hypothesis, the inventors first analyzed Myosin II and Ii distribution in CatS^{-/-} DCs by immunofluorescence. Theu chose to perform this analysis using DCs plated on rectangular fibronectin-coated micro-patterns to obtain a spatial intracellular resolution close to the one of elongated DCs migrating along micro-channels (Sup.Fig.6A). Non-activated WT DCs did not show a strong co-localization between Ii and Myosin II heavy chain (MyoIIHC). In these cells, Ii was rather heterogeneous: it was found in the perinuclear region and concentrated at one or at both cell poles. In both cases, a fraction of Ii-containing structures was consistently observed in close apposition with Myosin II (Fig.4A and Sup.Fig.6B). Noticeably, the inventors observed an enrichment of Myosin II staining in the Ii-containing structures that are known to accumulate in the perinuclear region of CatS^{-/-} DCs (Fig.4A) (*12*). Equivalent results were obtained in WT cells treated with the CatS inhibitor LHVS (Sup.Fig.6B). Cryoimmunoelectron microscopy experiments confirmed the presence of Myosin II light chain (MLC) in these enlarged dense endo-lysosomal compartments of CatS^{-/-} DCs (Fig.4B). Because these peculiar compartments form as a result of Ii retention in CatS^{-/-} cells (*16*) and have therefore no structural equivalent to be compared to in WT DCs, it was not possible to provide a rigorous quantification for this qualitative difference. However, we can state that we rarely found MLC and Ii co-localizing in endo-lysosomes of WT DCs (Sup.Fig.6C). This result was further verified quantitatively using biochemistry: the amounts of both MyoIIHC and MLC (20KDa) contained in semi-purified endosomal fractions were increased in CatS^{-/-} as compared to WT DCs (Sup.Fig.7A and 7B). Hence, retention of Ii in endo-lysosomes of CatS-deficient DCs promotes the association of Myosin II to these compartments.

When phosphorylated on Threonine 18 and/or Serine 19, MLC binds to and triggers the ATPase activity of the motor, initiating actomyosin contraction (*17*). To assess whether Myosin II retention in endo-lysosomes from CatS^{-/-} DCs decreases its activity, the inventors analyzed the phosphorylation of MLC. Phosphorylation of the full-length 20KDa MLC was considerably decreased in CatS^{-/-} cells as compared to their WT counterpart (Fig.4C and 4D for quantifications). This result indicates that the activity of Myosin II is indeed compromised in CatS^{-/-} DCs. Intriguingly, the inventors consistently observed that the extracts from CatS^{-/-} DCs contained a 12kDa protein that was recognized by the anti-MLC Ab but was not detected in WT cells (* on Fig.4C and Sup.Fig.7B). The identity of this polypeptide as corresponding to a fragment of MLC was confirmed by mass-spectrometry (Sup.Fig.7C, peptide identified in red). However, this MLC fragment did not react with the anti-phosphorylated MLC Ab (Fig.4D), maybe due to the lack of Threonine 18 and Serine 19 residues, suggesting that it is inactive. Strikingly, inhibition of CatS activity with LHVS triggered the accumulation of this 12kDa MLC fragment in WT but not in Ii^{-/-} DCs (Sup.Fig.7D). Hence, retention of Ii in CatS-deficient DCs induces the specific accumulation of a truncated form of MLC that to the inventors' knowledge has never been reported elsewhere. All together, these findings show that accumulation of Ii in DCs lacking CatS affects the sub-cellular localization and the activity of Myosin II, providing an additional biochemical evidence for a physical link between these two proteins and a possible mechanism for regulation of DC motility by Ii.

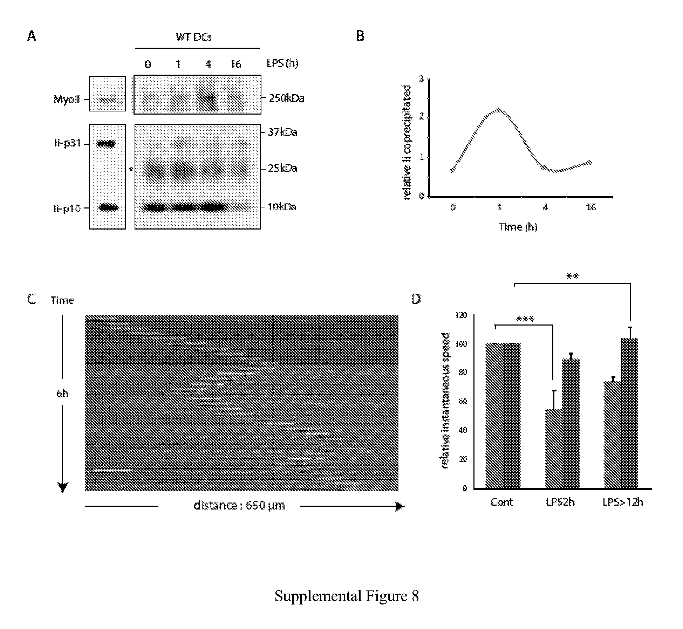

The inventors next investigated whether regulation of Ii expression upon DC activation leads to modification of their migratory capacity. Indeed, DCs undergo a transient increase in Ii and MHCII biosynthesis in the first hour after LPS-treatment (*4*, *18*). Accordingly, although Ii-Myosin II association was observed in non-activated DCs, it was further increased after 1h of LPS treatment (Sup.Fig.8A and B). The inventors found that WT DCs activated with LPS for 2h exhibited strong alterations in their ability to migrate along micro-channels. The effect of LPS was visualized at two levels: (1) early activated WT DCs displayed significantly reduced instantaneous velocities (Sup.Fig.8D), and (2) they displayed more fluctuant velocities and changed direction more frequently than untreated DCs, resulting in an even more pronounced reduction of mean velocities (Fig.4E and 4F, Sup.Fig.8C). The effect of LPS on motility was less marked in DCs treated with LPS during 12h, which moved slightly slower but with a similar directionality than non-activated DCs (Fig.4E and 4F). Importantly, LPS had no significant effect on the velocity and directionality of Ii-deficient DCs (Fig.4E, 4F, Sup.Fig.8D). The inventors conclude that the motility of DCs is regulated upon activation and that such regulation relies, at least in part, on Ii.

The inventors presently show that Ii acts as a brake during DC motion: it negatively regulates the migration of DCs by imposing a dis-continuous locomotion mode that alternates high and low motility phases. The present data further suggest that down-modulation of cell migration by Ii is likely to result from its direct or indirect association with Myosin II, which was shown to be required for polarized transport of MHCII-Ii complexes to endosomes in B cells (*7*). Ii-Myosin II interaction would facilitate the convergence between MHCII and the endocytosed Ag but would temporary affect the ability of Myosin II to promote cell migration, thus leading to a transient decrease in DC velocity (Fig.4G). Once in endosomes, Ii-Myosin II complexes would dissociate upon Ii cleavage by CatS, restoring high DC motility. This simple model explains all the present experimental observations: compromised Myosin II activity and cell migration in CatS-deficient DCs would result from Ii accumulation and permanent retention of a pool of Myosin II at the level of endosomes; in contrast, Ii-deficient DCs would display a higher and less fluctuant velocity than WT DCs due to constant devotion of Myosin II activity to cell motility.

The inventors propose that the use of common regulators for Ag processing and cell motility provides to DCs a way to coordinate these two functions in time and space. In immature DCs that patrol peripheral tissues, the periodic low motility phases induced by Ii enable DCs to efficiently couple Ag uptake/processing to cell migration, facilitating the sampling of the microenvironment. In early-activated DCs, the transient increase in the synthesis and association of Ii with Myosin II reduce the speed and confine the trajectories of the cells by promoting directional changes, enabling DCs to maximize Ag uptake (*2*). Interestingly, the inventors observed that the formation of endocytic vesicles at the front of migrating DCs occurs within defined motility phases, suggesting a possible coupling between macropinocytosis and cell locomotion.

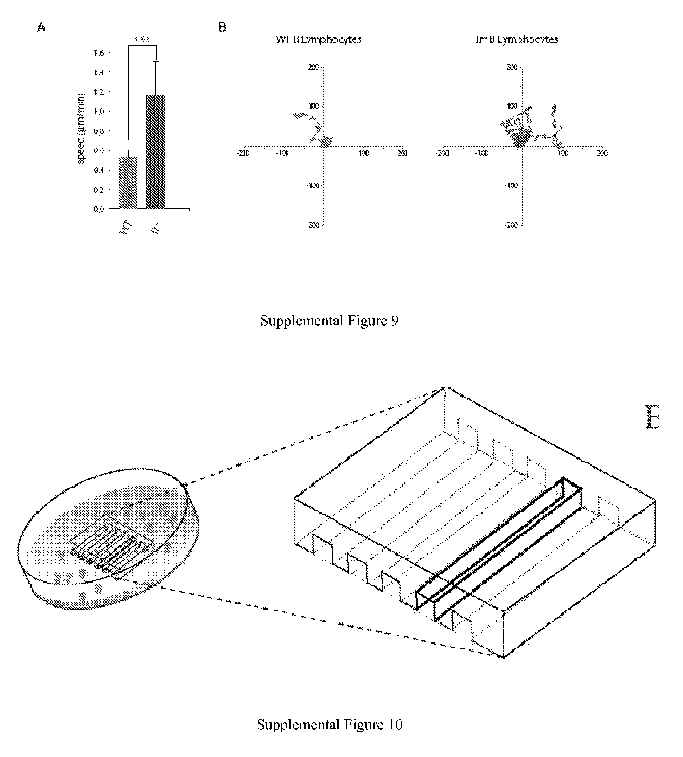

The physiological relevance of Myosin II-dependent migration in DCs and other leukocytes was recently demonstrated (*6*). The present work highlights a molecular mechanism used by DCs to regulate their migratory capacity via Myosin II. This mechanism is likely to be also utilized by B lymphocytes, in which Myosin II and Ii associate upon BCR engagement (*7*). Indeed, similarly to DCs, activated B cells from Ii^{-/-} mice display enhanced motility *ex vivo* (Sup.Fig.9A and B). Because Myosin II was also shown to respond to Ag receptors in T and NK cells (*19*) (*20, 21*), the inventors foresee that modulation of Myosin II activity might be a general mechanism used by leukocytes to regulate their specific immune function in time and space.

### Materials and methods

### Mice

Ii-deficient and CatS-deficient mice have been previously described (1, 2). C57BL/6 mice were purchased from Charles River Laboratories (France).

### Antibodies and Reagents

The following reagents were used: LPS (Sigma-Aldrich), Blebbistatin (Tocris) and LHVS (Calbichem No 219393). The following antibodies were used for biochemical experiments: rat anti-Ii IN-1 (BD biosciences), goat anti-CathepsinD (Santa Cruz Biotechnology), rat anti- tubulin (Serotec), rabbit anti-MLC (Cell Signalling Technologies), rabbit antiphosphorylated MLC (Rockland), rabbit anti-myosinIIA Heavy Chain (BTI), rabbit anti-Myosin II heavy chain (Covance), rabbit anti-I-A serum (JV2, a kind gift from Hidde Ploegh, Whiteshead Institute, MIT, USA), mouse anti-RhoA mAb 26C4 (a kind gift from Philippe Chavrier, UMR144, Institut Curie, France), biotin-anti-CD205 (Miltenyi Biotech), HRP-anti-rabbit IgG (Ozyme), HRP-anti-rat (Jackson). For cytofluorometry, streptavidin Alexa488 (Molecular Probes), PE-anti-CD86, APC-anti-CD11c, FITC-anti-I-A PE-anti-CD45.2, APC-anti-CD8a, and PE-anti-CD69 (BD biosciences) were used.

### Generation of Bone-marrow Dendritic cells

Mouse bone-marrow cells were cultured during 10-12 days in medium supplemented with fetal calf serum and granulocyte-macrophage colony stimulating factor-containing supernatant obtained from transfected J558 cells, as previously described (3). The phenotype of DCs was analyzed by cytofluorometry using a Facscalibur (BD Biosciences).

### Analysis of dendritic cell subsets in lymph nodes.

Cervical and inguinal LNs from WT and Ii_{-/-} mice were digested in 100 l of IMDM culture medium containing 400 U/ml of collagenase-D (Roche) plus 100 g/ml of DNAse-I (Roche) for 20 min at 37°C. Single cell suspensions were generated from digested tissues and stained using a mix of antibodies against CD11c, CD8 and CD205 diluted in PBS + 1% BSA. The mix was incubated 30min at 4°C and then analyzed by cytofluorometry after gating on the CD11c positive population.

### Migration of BM-DCs transferred in the footpad

DCs (10₆ cell/ml) were treated with LPS (10 g/ml, 10min) prior to labelling with either 5-(and 6)-carboxyfluorescein diacetate succinimidyl ester (CFSE) (Invitrogen) or 5-(and-6)-(((4-chloromethyl)benzoyl)amino)tetramethylrhodamine (CMTMR) (Invitrogen). Two different dyes were used to distinguish between WT and Ii- or CatS-deficient labeled DCs upon co-injection. Cells were washed twice in PBS-BSA 0,5% and resuspended in PBS-BSA 0,1% at 10x10₆ cells/ml, incubated with CFSE (5 M) or CMTMR (5 M) for 8 minutes at 37°C, and extensively washed with PBS-BSA 0,5%. Cells were resuspended in PBS at 10⁸ cells/ml, mixed 1:1 with the other DC type and then 30 l of the cell mix were injected in the footpad of recipient C57BL6 mice. 24h later, popliteal LNs were collected, treated with 1mg/ml of collagenase D (Roche) for 40 minutes at 37°C, mashed and analyzed by cytofluorometry. The percentage of migrated DCs corresponds to the ratio of fluorescent DCs to total LN cells.

### Skin sensitization experiments

Fluorescein (Sigma-Aldrich) was diluted to 1% in a 50/50 (vol/vol) acetone-dibutylphtalate mixture before application. The back of the ear of WT, CatS^{-/-} or Ii^{-/-} mice was painted with 20 l on this mixture, cervical LNs collected 24h later, treated with 1mg/ml of collagenase D (Roche) for 40 minutes at 37°C, mashed, labelled with an APC-conjugated anti-CD11c Ab and analyzed by flow cytometry. The percentage of migrated DCs corresponds to the ratio of CD11c+/FITC+ DCs to total LN cells.

### Transwell experiments

Uncoated transwells (Costar, 3 m pores) were placed in 24-well plates filled with 0,6ml of DC culture medium containing either nothing or a mixture of CCL19 and CCL21 chemokines (500ng/ml of each) (R&D). Overnight LPS-treated DCs (10 g/ml) were detached with PBS-EDTA 5mM for 5min at 37°C, counted and diluted in complete medium to reach a concentration of 10₅ cells/ml. 100 l of this solution were added to the upper side of the transwells and incubated at 37°C for 2h. Then the cells that reached the lower part of the transwell were counted. Experiments using collagen-I coated transwells (Becton Dickinson Labware) were performed similarly except that 3.10₅ untreated DCs were used and allowed to migrate during 24hours in the presence of LPS. For supernatant transfer experiments, fresh DCs were resuspended in the supernatant of LPS-treated DCs (O/N) and then added to the transwells.

### Sh-RNA knock-down experiments

Five different shRNA sequences for CD74 or Ii (NM_010545) were purchased (Sigma-Aldrich, ref: SHVRS-NM_004355) :
CCGGCCACACAGCTACAGCTTTCTTCTCGAGAAGAAAGCTGTAGCTGTGTGGTTTTT;
CCGGCGCGACCTTATCTCCAACAATCTCGAGATTGTTGGAGATAAGGTCGCGTTTTT;
CCGGCCACCGAAAGAGTCACTGGAACTCGAGTTCCAGTGACTCTTTCGGTGGTTTTT;
CCGGCCACCAAGTATGGCAACATGACTCGAGTCATGTTGCCATACTTGGTGGTTTTT;
CCGGGACCATAGACTGGAAGGTCTTCTCGAGAAGACCTTCCAGTCTATGGTCTTTTT.

These sequences were inserted in the pLKO.1/puromycin plasmid and transformed in *E.Coli* for propagation. Purified shRNA plasmids were then used to generate lentiviral particles by expressing it together with a *gag-pol-env*-encoding plasmid in the HEK 293T packaging cell line. Centrifuged culture supernatants (4ml) containing lentivirus particles were used to infect day 4 BM-DCs. On day 7, Puromycin (4 g/ml) was added to the culture to select the cells that have been efficiently infected by the viral particles, as the shRNA transgene encodes the puromycin-resistance gene. Culture medium was replaced on day 8. On day 10, DCs were collected, washed three times with PBS and used either to prepare protein extracts for immunoblotting (25 g of proteins per lane loaded on the gel) or to analyze their migration in collagen-coated transwells, as described above.

### Immunoblotting and co-immunoprecipitation experiments

DCs were lysed in (100mM Tris, 150mM NaCl, 0,5% NP-40, and 5% protease cocktail inhibitors), cell lysates quantified and 15-50mg of extracts mixed with reducing laemmli buffer, boiled and loaded onto a 4-12% SDS-PAGE gradient gel (Invitrogen). Proteins were transferred onto a PVDF membrane (Immobilon-P, Millipore), the membrane incubated with the appropriate Ab and revealed with ECL (GE Healthcare). For co-immunoprecipitation, BM-DCs were or not treated with 1 g/ml of LPS during 30min, 1h or 4h. Cells were lysed as described above and 800µg of cell lysates was pre-cleared once with rabbit and mouse non-immune sera with protein G-coated Sepharose beads and once with protein G-coated Sepharose beads alone. Myosin II was immunoprecipitated using 2 g of rabbit antibody Myosin II heavy chain antibody (Covance). Samples were washed, resuspended in reducing buffer, boiled, and loaded onto a 4-12% SDS-PAGE gradient gel. Protein transfer to PVDF membranes and immunoblot analysis were performed as described above.

### Endosome purification

Endosomes from BM-DCs were purified by magnetic fractionation as already described for B cells (4). Briefly, BM-DCs were resuspended in cold culture medium at 25.10₆ cells/ml and incubated for 30min on ice with magnetic nanoparticles (15 M, Fe₂O₃ coated with BSA, kindly provided by J. Roger, Paris VI University, Paris, France). After the pulse, cells were incubated at 37°C for 60min to allow nanoparticles to be internalized and then incubated at 0°C to stop endosome maturation. Cells were washed three times with cold PBS and resuspended in homogenization buffer (Imidazol 3mM/sucrose 8%/DTT 1mM/EDTA 1mM/protease inhibitors). DCs were broken by flushing them 10 times through a 25G needle. Nuclei and intact cells were removed by centrifugation (750g, 10 min, 4°C) and the postnuclear supernatant (PNS) was collected and placed on a magnet (Dynal) O/N at 4°C. Non-retained material was eluted (non magnetic fraction, NMF). Retained material (magnetic fraction, MF) was collected in 50 l of homogenization buffer. Both the NMF and MF were analyzed by immunoblotting performed on 12% SDS-PAGE gels (Invitrogen).

### Gelatin-FITC degradation

Gelatin-FITC (Invitrogen) was dissolved in 25ml of PBS with 2% sucrose and centrifuged 5min at 4500rpm to pellet non-dissolved fragments. EtOH-washed coverslips were coated with poly-L-lysine at 50 g/ml in PBS for 20min at RT, washed, fixed with glutaraldehyde 0,5% and washed again. Gelatin-FITC was then added to coverslips for 10min at RT in the dark, washed with PBS followed by sodium borohydride (NaBH4, 5 g/ml, 3min) and then with PBS again. DCs were allowed to adhere on the gelatin-coated coverslips during 10min in PBS at RT and incubated at 37°C in culture medium containing LPS (10 g/ml) during 6h. After washing and fixation with paraformaldehyde 4%, DCs were stained with phalloidin-fluoProbes 547 and DAPI (Molecular Probes). To quantify degradation, the area of each degraded patch was measured using the Metamorph software. For each condition the total area of degradation was normalized to the total cell number on the slide (also quantified with Metamorph) and expressed relative to the WT control.

### Immunofluorescence on micropatterns

Line shape fibronectin-coated micro-patterns (50x10 M) were fabricated as already described (5). Immature DCs were cultured 45min on the patterned slides in order to allow adhesion and non adhered cells were washed out with culture medium. The slides were further cultured for an additional 45min in complete medium at 37°C to allow cell polarization. Cells were fixed in PBS + 4%PFA for 10min at RT. Cells were permeabilized by incubation at RT for 20min with PBS + 0,2%BSA + 0,05%saponin. Slides were stained with rat-anti-Ii (1/100) and rabbit-anti-Myosin II (1/500) Abs followed by a mix of anti-rat Alexa488 and anti-rabbit Alexa647 antibodies (Molecular Probes). All washes and Ab dilutions were performed in permeabilization buffer. Slides were washed, mounted and analyzed on a Zeiss confocal microscope (LSM Axiovert 720) with a 63x 1.4 NA oil immersion objective.

### Cryoimmunoelectron microscopy

2-4x10⁶ of 1h-LPS-treated BM-derived DCs processed for cryoimmunoelectron microscopy as previously described (*6*).

### Proteomics Analysis

Ii was immunoprecipitated from 1mg of a DC protein extract as previously described (*4*). Immunoprecipitates were separated on a 4-12% SDS-PAGE gradient gel (Invitrogen), and 1-2mm gel fragments were cut around the 12kDa region. The fragments were washed by adding NH₄HCO₃ (25mM) and acetonitrile and then dried. Proteins were reduced with DTT (10mM) during one hour at 57°C and alkylized with iodoacetamide (55mM) one hour in the dark. After washing with NH₄HCO₃ and acetonitrile, samples were digested with trypsin (12,5ng/ml) during 16h at 30°C. Peptides were extracted with a mixture of acetonitrile/HCOOH/H20 (60/5/35 vol.) and analyzed by MS/MS on a QSTAR/pulsar*i* (from Applied Biosystems, Proteomics facility, Curie Institute).

### Migration in micro-channels

The microfluidic device was fabricated in PDMS using rapid prototyping and soft lithography (*7*). The PDMS piece, with embedded microchannels and holes for the inlet and outlet ports, and a glass Iwaki chamber (Milian) were activated in a plasma cleaner (PDC-32G Harrick) for 30s and bonded to each other. The chambers were left under strong vacuum for 5min in the plasma cleaner and plasma was turned on for 10s to render the top surface of the PDMS and the inlet and outlet holes hydrophilic. Drops of fibronectin solution at 50µg/ml were placed on top of the inlet and outlet ports. The solution spontaneously invaded the channels and all air bubbles were resorbed into the PDMS due to the previous vacuum treatment. Fibronectin was incubated for 1h at room temperature and then replaced first by PBS then by cell culture medium.

The cells were concentrated and micropipette tips containing the cells were inserted in the inlet port. Cells fell in the port by gravity, bound to the bottom coverslip and started migrating. They entered the channels spontaneously, without any mechanical or chemical stimulation. Phase contrast images at various positions in the chambers were recorded with time-lapses of 1s (for single cell at high resolution) to 2min (to record multiple fields at low resolution for statistics), during 1-12h, using an automated microscope (Nikon ECLIPSE TE1000-E, and Olympus X71, with a Marzhauser motorized stage and an HQ2 Roper camera) equipped with an environmental chamber for temperature, humidity and CO2 (Life Imaging Services). Cells remained alive and motile during the entire period of recording.

To assess the effect of LPS on DC migration into micro-channels, day 12 BM-derived DCs were collected and suspended in medium with or without LPS (10 g/ml). They were immediately introduced in fibronectin-coated micro-channels previously filled with medium containing or not LPS at the same concentration. 1h30 to 2h following introduction of the cells, phase contrast images were recorded with time-lapses of 2min during 12h as described in the previous paragraph.

### Film analysis

**Kymograph extraction:** A program written in C++, taking as input an image sequence, provides as output a set of kymographs corresponding to each channel by automatically performing motion compensation, background subtraction, channels detection and kymographs computation, without any intervention from the user. A number of parameters are accessible when the program is started. Each resulting kymograph is an image which contains, in each horizontal line, the grey values found along a given channel at a given time point. Then the consecutive time points form the consecutive lines of the image, with time zero at the top. This enables to reduce large data sets (thousands of images) into a smaller set of images (one per channel), which contains all the necessary information for cell movement analysis. Basically, the space dimension perpendicular to the channels length, which contains no information, is suppressed. The program first performs image cleaning: indeed, at 10x magnification, with phase contrast microscopy, 4µm wide channel display a strong contrast and cells in the channels are hardly visible on original images (see supplemental movies). Moreover, due to fast movements of the stage in order to get enough positions recorded at a high temporal rate, image sequences display slight giggling, due to re-positioning errors. Sub-pixel phase correlation (8) and robust multi-resolution estimation of translation motion model (9) is used for registration. Then background subtraction is done before the computation of the kymograph. The background is estimated by taking the average intensity along the image sequence. To produce kymographs, first, channels were detected using the Hough transform (10). An average width for the lines is defined as the half of the distance between two channels (this parameter can be varied). Intensities of the kymographs are then defined as the maximum intensity inside the bound of the line encountered perpendicularly to it.

**Instantaneous velocities analysis:** Kymographs were then analysed using homemade routines in Matlab. Cell signature was identified in each line and the cells center of mass and boundaries were found. Statistics and graphs were extracted from the data using Matlab.

### B lymphocyte motility assays

A single-cell suspension was generated by mechanical disruption of spleens from 8-12 week-old mice and resting mature IgM+/IgD+ B cells were purified by negative selection as previously described (4). B cells were plated on poly-L-lysine-coated 35mm glass slides at 37°C in the presence of BCR multivalent ligand (10 µg/ml F(ab')2 goat antimouse IgM cross-linked with 20 µg/ml F(ab')2 donkey anti-goat IgG) (4). Phase contrast images at various positions were recorded with a 40x objective every minute during 2h and mean velocities were calculated by tracking each cell movement using the Metamorph software. 48 cells were tracked per condition and per experiment and two independent experiments were performed.

### REFERENCES

1. J. A. Villadangos, P. Schnorrer, N. S. Wilson, Immunol Rev 207, 191 (Oct, 2005).
2. M. A. West et al., Science 305, 1153 (Aug 20, 2004).
3. P. Pierre et al., Nature 388, 787 (Aug 21, 1997).
4. M. Cella, A. Engering, V. Pinet, J. Pieters, A. Lanzavecchia, Nature 388, 782 (Aug 21, 1997).
5. C. Reis e Sousa, Nat Rev Immunol 6, 476 (Jun, 2006).
6. T. Lammermann et al., Nature, (2008).
7. F. Vascotto et al., J Cell Biol 176, 1007 (Mar 26, 2007).
8. M. F. Naujokas, M. Morin, M. S. Anderson, M. Peterson, J. Miller, Cell 74, 257 (Jul 30, 1993).
9. X. Shi et al., Immunity 25, 595 (Oct, 2006).
10. G. P. Shi et al., Immunity 10, 197 (Feb, 1999).
11. T. Y. Nakagawa et al., Immunity 10, 207 (Feb, 1999).
12. C. Driessen et al., J Cell Biol 147, 775 (Nov 15, 1999).
13. R. J. Riese et al., J Clin Invest 101, 2351 (Jun 1, 1998).
14. S. H. Chao, R. Carlson, D. R. Meldrum, Lab Chip 7, 641 (May, 2007).
15. D. Irimia, G. Charras, N. Agrawal, T. Mitchison, M. Toner, Lab Chip 7, 1783 (Dec, 2007).
16. M. Boes et al., Nature 418, 983 (Aug 29, 2002).
17. K. Clark, M. Langeslag, C. G. Figdor, F. N. van Leeuwen, Trends Cell Biol 17, 178 (Apr, 2007).
18. J. A. Villadangos et al., Immunity 14, 739 (Jun, 2001).
19. J. Jacobelli, S. A. Chmura, D. B. Buxton, M. M. Davis, M. F. Krummel, Nat Immunol 5, 531 (May, 2004).
20. M. M. Andzelm, X. Chen, K. Krzewski, J. S. Orange, J. L. Strominger, J Exp Med 204, 2285 (Oct 1, 2007).
21. K. Krzewski, X. Chen, J. S. Orange, J. L. Strominger, J Cell Biol 173, 121 (Apr 10, 2006).

## Claims

1. CD74 modulator agent for regulating the dendritic cell migration.

2. CD74 modulator agent according to claim 1, wherein the CD74 modulator agent is a CD74 inhibitor for increasing the dendritic cell migration.

3. CD74 modulator agent according to claim 1, wherein the CD74 modulator agent is a CD74 activator for decreasing the dendritic cell migration.

4. CD74 modulator agent according to claim 2, wherein the CD74 inhibitor is selected from the group consisting of a MIF inhibitor, an antibody against CD74, a CD74 antagonist, a soluble CD74, and an antisense, siRNA, shRNA and the like decreasing the expression of CD74.

5. CD74 modulator agent according to claim 3, wherein the CD74 activator is selected from the group consisting of a CatS inhibitor, a CD74 agonist, MIF and a MIF agonist.

6. CD74 modulator agent according to anyone of claims 1-5 for treating or preventing auto-immune diseases, cancers, inflammatory diseases or viral infections.

7. A device for determining or studying the motility capacity of a cell, wherein the device comprises at least one embedded micro-channel with holes for the inlet and outlet ports, said micro-channel having diameter smaller than the cell diameter.

8. Use of a device according to claim 7 for determining or studying the motility capacity of a cell.

9. A method for determining or studying the motility capacity of a cell, comprising introducing the cell into the inlet port of the device of claim 7 and measuring the move of the cell into the micro-channel.
